# FASCICULE DE BREVET EUROPEEN

(11) **EP 3 990 279 B1**
(45) Date de publication et mention de la délivrance du brevet: **26.07.2023**
(21) Numéro de dépôt: 20747042.8
(22) Date de dépôt: 25.06.2020
(51) Int. Cl.: B32B 27/08, B32B 27/32, A61J 1/00, A61L 2/08

(54) **CONDITIONNEMENTS POLYMÉRIQUES ET LEUR UTILISATION POUR CONSERVER UNE COMPOSITION PHARMACEUTIQUE**
POLYMERVERPACKUNG UND IHRE VERWENDUNG ZUR KONSERVIERUNG EINER PHARMAZEUTISCHEN ZUSAMMENSETZUNG
POLYMER PACKAGING AND USE THEREOF FOR PRESERVING A PHARMACEUTICAL COMPOSITION

(30) Priorité: 26.06.2019 FR 1906965
(43) Date de publication de la demande: 04.05.2022
(73) Titulaire: Ceva Santé Animale, 33500 Libourne (FR)
(72) Inventeur: LACOSTE, Sandrine, 33500 LIBOURNE (FR); GUIMBERTEAU, Florence, 33500 LIBOURNE (FR)
(74) Mandataire: Cabinet Becker et Associés
(86) Numéro de dépôt international: PCT/FR2020/051112
(87) Numéro de publication internationale: WO 2020/260833

(56) Documents cités:
- EP-A1- 0 679 506
- EP-A1- 1 033 326
- US-A- 4 959 271
- US-A1- 2010 176 136

## Description

### OBJET DE L'INVENTION

La présente invention concerne le domaine des polymères, et en particulier, celui des conditionnements polymériques, tels que des flacons, pouvant être utilisés pour conserver des compositions pharmaceutiques stériles.

### ARRIERE-PLAN TECHNOLOGIQUE DE L'INVENTION

La stérilisation est une opération très importante, voire essentielle, dans les processus de fabrication de compositions pharmaceutiques. En effet, la plupart des compositions pharmaceutiques doit être fabriquée et conservée dans des conditions stériles avant d'être administrée chez le patient pour éviter l'introduction de germes pathogènes, et conserver une stabilité chimique et physique optimale. L'étape de stérilisation, dans un processus de conservation stérile d'une composition pharmaceutique, peut être réalisée soit sur l'ensemble conditionnement-composition pharmaceutique ou sur chacun d'entre eux pris isolément suivi d'un remplissage du conditionnement avec la composition pharmaceutique, le tout en milieu stérile.

L'Agence Européenne des Médicaments (EMA) a récemment publié de nouvelles directives (EMA/CHMP/CVMP/QWP/850374/2015) sur les conditions à respecter pour la stérilisation des produits médicinaux et vétérinaires, incluant les compositions pharmaceutiques (substances actives et excipients), mais aussi les conditionnements contenant ces compositions pharmaceutiques. Dans la mesure du possible, ces directives préconisent de réaliser l'étape de stérilisation terminale de la composition pharmaceutique, lorsque celle-ci est présente, dans son conditionnement final.

Il existe différents types de méthodes couramment utilisées pour stériliser un conditionnement vide, c'est-à-dire sans composition pharmaceutique à l'intérieur, ou un conditionnement contenant une composition pharmaceutique. On peut citer, par exemple, les procédés de stérilisation par chaleur humide ou sèche, les méthodes par irradiation, et les procédés par stérilisation gazeuse qui doivent être mis en oeuvre en dernier lieu lorsque les procédés par chaleur ou irradiation ne sont pas adaptés. Les directives préconisent des cycles de plusieurs minutes réalisés à des températures comprises entre 110 et 121 °C pour les procédés de stérilisation par chaleur humide. Pour la mise en oeuvre des procédés de stérilisation par chaleur sèche, une durée d'au moins deux heures à une température minimum de 160 °C est préconisée. En ce qui concerne les procédés de stérilisation par irradiation, la dose d'irradiation de référence est au minimum de 25 kGy.

Ces différents procédés de stérilisation requièrent ainsi des conditions particulières pour obtenir une composition pharmaceutique stérile optimale, lesquelles doivent être bien supportées par le conditionnement contenant la composition pharmaceutique. Plus spécifiquement, le conditionnement doit présenter des propriétés de résistance, de stabilité, de résistance mécanique ou de robustesse, de transparence, d'imperméabilité aux facteurs environnementaux (lumière, oxygène, température), pendant et après le processus de stérilisation, notamment de manière à pouvoir conserver une composition pharmaceutique stérile le plus longtemps possible. Ce conditionnement pharmaceutiquement acceptable ne doit pas non plus altérer lui-même la qualité des compositions pharmaceutiques qu'il contient. Inversement, la composition pharmaceutique ne doit pas altérer la qualité de ce conditionnement.

Le document US2010/176136 A1 décrit un récipient multicouche stérilisable, pour la conservation d'une composition pharmaceutique, comprenant une couche extérieure en contact direct avec l'environnement, qui comprend des polyoléfines.

Il existe ainsi aujourd'hui un besoin de développer et fournir de nouveaux conditionnements résistants à des conditions de stérilisation de plus en plus fortes ou exigeantes, telles que celles préconisées par les nouvelles directives de l'EMA, et permettant de conserver durablement des compositions pharmaceutiques stables et stériles.

### RESUME DE L'INVENTION

Devant cette problématique, les inventeurs proposent un nouveau conditionnement résistant pour la conservation ou le stockage d'une composition pharmaceutique stérile.

Ce conditionnement comprend notamment une couche externe polymérique particulière permettant à ce conditionnement de supporter les conditions de stérilisation recommandées par les directives de l'EMA tout en conservant sa robustesse. Plus spécifiquement, les inventeurs ont démontré, de manière surprenante, qu'un conditionnement comprenant au moins une couche externe polymérique en polyéthylène d'une épaisseur comprise entre 300 et 1000 µm conservait toute sa résistance lorsqu'il était soumis à de fortes doses d'irradiations (autour de 40 kGy), permettant ainsi de conserver durablement une composition pharmaceutique stérile.

La présente invention concerne donc un conditionnement, en particulier pour la conservation d'une composition pharmaceutique stérile, comprenant au moins une couche interne de polypropylène ayant une épaisseur comprise entre 200 et 800 µm et une couche externe de polyéthylène, ladite couche externe étant en contact avec l'environnement et ayant une épaisseur comprise entre 300 et 1000 µm.

Selon un mode particulier, la couche interne de polypropylène a une épaisseur comprise entre 300 et 700 µm, entre 350 et 650 µm, entre 400 et 600 µm, de préférence entre 450 et 550 µm, et d'une manière encore plus préférée d'environ 500 µm.Selon un autre mode particulier, la couche externe de polyéthylène a une épaisseur comprise entre 400 et 800 µm, entre 450 et 750 µm, entre 500 et 700 µm, de préférence entre 550 et 650 µm, et d'une manière encore plus préférée d'environ 600 µm. Selon un mode préféré, la couche externe de polyéthylène comprend en outre au moins un colorant anti-UV.

Selon un autre aspect de l'invention, le conditionnement comprend en outre une couche centrale d'agent barrière aux gaz située entre la couche interne de polypropylène et la couche externe de polyéthylène, et ayant une épaisseur comprise entre 10 et 250 µm, entre 10 et 200 µm, entre 20 et 80 µm de préférence entre 20 et 50 µm, et d'une manière encore plus préférée d'environ 30 ou 50 µm. De préférence, la couche centrale d'agent barrière aux gaz comprend de l'éthylène-alcool vinylique.

Un conditionnement particulier de l'invention comprend ainsi au moins les couches suivantes :
- une couche interne de polypropylène ayant une épaisseur d'environ 500 µm,
- une couche centrale comprenant de l'éthylène vinyle alcool ayant une épaisseur d'environ 30 ou 50 µm, et
- une couche externe de polyéthylène ayant une épaisseur d'environ 600 µm.

De préférence, ce conditionnement comprend deux couches adhésives intercalaires ayant chacune une épaisseur comprise entre 5 à 50 µm, de préférence d'environ 10 µm, lesdites deux couches adhésives intercalaires étant respectivement situées entre la couche interne de polypropylène et la couche centrale d'agent barrière aux gaz, et entre la couche externe de polyéthylène et la couche centrale d'agent barrière aux gaz.

Selon un mode préféré de l'invention, le polyéthylène est un polyéthylène basse densité.

La présente invention concerne également un conditionnement tel que décrit dans la présente demande contenant en outre une composition pharmaceutique stérile. De préférence, la composition pharmaceutique stérile est une composition aqueuse ou non-aqueuse. Selon un mode préféré, la composition non-aqueuse est une composition huileuse. Particulièrement, la composition pharmaceutique stérile comprend un principe actif choisi parmi l'amoxicilline, la buséréline, le toltrazuril, le diclazuril, ou la céfalexine.

Selon un mode préféré de l'invention, le conditionnement est une bouteille ou un flacon.

Un autre objet de l'invention porte sur une utilisation d'un conditionnement, tel que décrit dans la présente demande, pour conserver une composition pharmaceutique stérile.

Un autre objet de l'invention concerne un procédé pour la conservation stérile d'une composition pharmaceutique comprenant les étapes suivantes :
a) ajouter une composition pharmaceutique dans un conditionnement tel que décrit dans la présente demande ; et
b) stériliser le conditionnement contenant la composition pharmaceutique.

Selon un mode préféré, le conditionnement contenant la composition pharmaceutique à l'étape b) est stérilisé par irradiation gamma, de préférence à une dose comprise entre 15 et 40 kGy.

### DESCRIPTION DETAILLEE DE L'INVENTION

La présente invention fournit un conditionnement permettant de conserver, sur le long-terme, une composition pharmaceutique stérile. Plus particulièrement, ce conditionnement présente l'avantage d'être résistant et stable aux conditions de stérilisation, en particulier aux fortes doses d'irradiation gamma, de pouvoir être imperméable à l'oxygène et à l'eau, et de conserver sa transparence.

Tel qu'utilisé dans la présente demande, le terme environ sera compris par l'homme du métier et peut varier dans une certaine mesure selon le contexte dans lequel il est utilisé. Si certaines utilisations de ce terme ne sont pas claires pour l'homme du métier en fonction du contexte, « environ » signifie plus ou moins 20 %, de préférence plus ou moins 10 % de la valeur spécifiée.

### Conditionnement

La présente invention concerne un conditionnement, en particulier pour la conservation d'une composition pharmaceutique stérile, comprenant au moins une couche interne de polypropylène ayant une épaisseur comprise entre 200 et 800 µm et une couche externe de polyéthylène, ladite couche externe étant en contact avec l'environnement et ayant une épaisseur comprise entre 300 et 1000 µm.

Selon l'invention, le conditionnement comprend au moins une couche interne de polypropylène.

Par « couche interne », on entend la couche située à l'intérieur du conditionnement. Elle correspond ainsi à la couche en contact avec la composition pharmaceutique lorsque celle-ci est présente dans le conditionnement.

Le polymère polypropylène (ou polypropène), désigné également dans la présente demande sous le sigle « PP », a pour formule chimique -(CH₂-CH(CH₃))ₙ-. Le polypropylène résulte de la polymérisation coordinative des monomères propylènes (CH₂=CH-CH₃) en présence de catalyseurs tels que ceux utilisés dans le procédé de Ziegler-Natta. Le polypropylène présente une densité autour de 0,9 g/cm³, plus précisément comprise entre 0,895 et 0,920 g/cm³ et un module de Young, également appelé module d'élasticité ou de traction, compris entre 1,1 et 1,6 Gpa (gigapascal). Il existe trois différents types de polypropylène. Le premier type comprend uniquement des monomères de propylène sous une forme solide semi-cristalline et est appelé « homo-polymère de polypropylène ». Le second type correspond à des copolymères statistiques propylène/éthylène. Ils comprennent, en plus des monomères de propylène, une quantité molaire de 1 à 8% de monomères d'éthylène. Enfin, le troisième type correspond à un homo-polymère de polypropylène comprenant en outre une phase de copolymères statistique propylène/éthylène ayant une quantité molaire de 45 à 65% de monomères d'éthylène. On les appelle des copolymères choc de polypropylène. Des polypropylènes commercialement disponibles sont par exemple le polypropylène Aceso^{®} PPM R021 de Total et le polypropylène BORMED SB815MO de Borealis. Selon un mode préféré de l'invention, le polypropylène formant la couche interne du conditionnement est un copolymère statistique propylène/éthylène comprenant une quantité molaire de 3% de monomères d'éthylène.

Selon l'invention, la couche interne du conditionnement en polypropylène a une épaisseur comprise entre 200 et 800 µm. Plus particulièrement, la couche interne de polypropylène a une épaisseur comprise entre 300 et 700 µm, entre 350 et 650 µm, entre 400 et 600 µm, 420 et 580 µm, entre 430 et 570 µm, entre 440 et 560 µm, entre 450 et 550 µm, entre 460 et 540 µm, entre 470 et 530 µm, entre 480 et 520 µm, entre 490 et 510 µm, et de préférence d'environ 500 µm.

Selon l'invention, le conditionnement comprend au moins une couche externe de polyéthylène.

Par « couche externe », on entend la couche située à la périphérie du conditionnement. Elle correspond ainsi à la couche en contact avec l'environnement.

Le polyéthylène, désigné également dans la présente demande sous le sigle « PE », a pour formule chimique -(CH₂-CH₂)ₙ-. Il résulte de la polymérisation radicalaire des monomères d'éthylène (CH₂=CH₂) sous très haute pression (1800 à 3000 bars) et à des températures autour de 200°C. Il existe différents types de polyéthylènes dont les polyéthylènes basse densité (« PEBD »), les polyéthylènes linéaires base densité (« PEBDL »), les polyéthylènes moyenne densité (« PEMD »), et les polyéthylènes haute densité (« PEHD »).

Comme son nom l'indique, le PEBD présente une densité voisine de 0,92 g/cm³, plus précisément comprise entre 0,895 et 0,925 g/cm³, plus faible que la densité du polyéthylène moyenne densité (PEMD) (0,926-0,940 g/cm³) et du polyéthylène haute densité (PEHD) (0,941-0,965 g/cm³) qui s'explique par son taux de ramification supérieur à ceux du PEMD et du PEHD. Le PEBD présente un module de Young compris entre 0,1 et 0,3 Gpa. Le PEBD peut être soit d'origine pétrolière ou fossile, ou d'origine végétale. Des PEBD d'origine fossile sont, par exemple, le PEBD « Bormed^{™} LE6607-PH » commercialisé par la société Borealis et le PEBD « *Purell* PE 3020 D » commercialisé par la société LyondellBasell. Le PEMD présente un module de Young de 172 à 379 Mpa. Des PEMD disponibles sur le marché, sont par exemple, les PEMD de la gamme Lumicene^{®} commercialisés par la société Total. Le PEHD présente un module de Young compris entre 0,5 et 1,2 Gpa. Le PEHD peut être soit d'origine pétrolière ou d'origine végétale. Un PEHD d'origine végétale ou « biosourcé », comme tout matériaux biosourcés, a pour effet réduire l'empreinte écologique ou environnementale. Des PEHD d'origine fossile sont, par exemple, les PEHD commercialisés par la société Sabic, tels que HDPE BM6246LS. Un PEHD d'origine végétale ou biosourcé est, par exemple, le PEBD « SGF 4950 » commercialisé par la société Braskem.

Selon un mode préféré de l'invention, le polyéthylène est un polyéthylène basse densité.

Selon un mode particulier de l'invention, le polyéthylène basse densité est d'origine végétale. De préférence, le PEBD est produit à partir de plantes, telles que la canne à sucre. L'utilisation d'un tel PEBD « biosourcé » dans la couche externe du conditionnement a pour effet de réduire l'empreinte écologique ou environnementale du conditionnement, par rapport à l'utilisation d'un PEBD d'origine fossile. Un PEBD particulier « biosourcé » ou d'origine végétale est, par exemple, le PEBD « STN7006 » commercialisé par la société Braskem.

Selon l'invention, la couche externe du conditionnement en PE a une épaisseur comprise entre 400 et 800 µm. Plus particulièrement, la couche externe de PE a une épaisseur comprise entre 450 et 750 µm, entre 500 et 700 µm, entre 550 et 650 µm, entre 560 et 640 µm, entre 570 et 630 µm, entre 580 et 620 µm, entre 590 et 610 µm, et de préférence d'environ 600 µm.

Un conditionnement préféré de l'invention pour la conservation d'une composition pharmaceutique stérile comprend au moins une couche interne de polypropylène ayant une épaisseur comprise d'environ 500 µm et une couche externe de polyéthylène, de préférence de polyéthylène basse densité, ladite couche externe étant en contact avec l'environnement et ayant une épaisseur comprise d'environ 600 µm.

Selon un mode particulier, la couche externe de polyéthylène, de préférence de polyéthylène basse densité, du conditionnement comprend en outre au moins un additif. Ces additifs sont par exemple des antioxydants, des plastifiants, des stabilisants, des lubrifiants, des colorants (ou « DYE »), ou des renforceurs mécaniques. De préférence, la couche externe de polyéthylène du conditionnement comprend en outre au moins un colorant, de préférence avec des propriétés anti-UV. Selon un mode encore plus préféré, la couche externe de polyéthylène du conditionnement comprend un colorant avec des propriétés anti-UV. Ce conditionnement comprenant une couche externe comprenant en outre au moins un ou un colorant anti-UV est particulièrement adapté à la conservation d'une composition pharmaceutique stérile sensible aux rayons UV. Selon un mode particulier, le colorant anti-UV est présent dans une proportion inférieure ou égale à 5% en poids, par rapport au poids total de la couche externe, en particulier 5%, 4%, 3%, 2%, 1%, et de manière préférée dans une proportion de 3% en poids, par rapport au poids total de la couche externe. Des colorants sont, par exemple, les produits de la gamme Colorant Cleartint^{™} commercialisés par la société Milliken, les produits de la gamme Mevopur^{®} commercialisés par la société Clariant, et les produits commercialisés par le groupe Gabriel-Chemie, tels que la référence HP 79860 UV.

Selon un mode particulier de l'invention, le conditionnement comprend en outre une couche centrale d'agent barrière aux gaz. Ce conditionnement comprenant cette couche centrale d'agent barrière aux gaz est particulièrement adaptée à la conservation d'une composition pharmaceutique stérile sensible aux gaz, en particulier sensible à l'oxygène. Par « couche centrale d'agent barrière aux gaz », on entend une couche centrale d'agent barrière aux gaz située entre la couche interne de polypropylène et la couche externe de polyéthylène basse densité. De par sa position, la couche centrale n'est donc ni en contact avec l'environnement, ni en contact avec la composition pharmaceutique stérile lorsque celle-ci est présente dans le conditionnement. De préférence, la couche centrale d'agent barrière aux gaz a une épaisseur comprise 10 et 250 µm, entre 10 et 200 µm, entre 20 et 80 µm, entre 30 et 70 µm, de préférence entre 20 et 50 µm, et d'une manière encore plus préférée d'environ 30 ou 50 µm. Selon un mode encore plus particulier de l'invention, le conditionnement comprend en outre une couche centrale d'agent barrière aux gaz située entre la couche interne de polypropylène et la couche externe de polyéthylène, et ayant une épaisseur comprise entre 10 et 250 µm, entre 10 et 200 µm, entre 20 et 80 µm, de préférence entre 20 et 50 µm, et d'une manière encore plus préférée d'environ 30 ou 50 µm.

De préférence, la couche centrale d'agent barrière aux gaz comprend de l'éthylène-alcool vinylique ou de l'acide polyglycolique, avantageusement de l'éthylène alcool vinylique.

L'éthylène-alcool vinylique ou éthylène vinyle alcool désigné également dans la présente demande sous le sigle « EVOH », est un copolymère ayant pour formule chimique -(CH₂-CHOH)ₓ-(CH₂-CH₂)_{y}. Il résulte de la polymérisation des monomères d'éthylène et des monomères vinyl acétate suivie d'une hydrolyse. Le copolymère EVOH est défini par la quantité de moles d'éthylène (%). Plus le pourcentage de moles d'éthylène est faible, meilleures sont les propriétés d'agent barrière aux gaz. Selon un mode préféré, l'EVOH comprend entre 20 et 60 %, entre 27 et 47 %, et d'une manière encore plus préférée, environ 32 % de moles d'éthylène. On peut citer comme exemples d'EVOH, les produits EVAL F101 et SOARNOL commercialisés respectivement par les sociétés KURARAY et GOSHEI.

L'acide polyglycolique désigné également dans la présente demande sous le sigle « PGA » est un polymère d'origine végétal, biodégradable et thermoplastique. Le PGA présente notamment des propriétés améliorées en ce qui concerne l'effet de barrière aux gaz, la résistance à la chaleur, la moulabilité, la transparence, et la durabilité. Un exemple de PGA commercial est le produit KUREDUX de la société KUREHA.

Un autre conditionnement préféré de l'invention, pour la conservation d'une composition pharmaceutique stérile, comprend au moins les couches suivantes :
- une couche interne de polypropylène ayant une épaisseur d'environ 500 µm,
- une couche centrale comprenant de l'éthylène vinyle alcool ayant une épaisseur d'environ 30 ou 50 µm, et
- une couche externe de polyéthylène ayant une épaisseur d'environ 600 µm.

Optionnellement, le conditionnement peut comprendre une ou plusieurs couches adhésives intercalaires pouvant être situées entre la couche interne de PP et la couche externe de PE, ou entre la couche interne de PP et la couche centrale d'agent barrière aux gaz et entre la couche externe de PE et la couche centrale d'agent barrière aux gaz.

La couche adhésive intercalaire comprend un agent adhésif formé à partir de polymères, tels que les polyéthylènes, les polypropylènes, les EVOHs, les polyamides, les polycarbonates, les polystyrènes, les polytéréphtalates et qui sont modifiés par des groupements fonctionnels adaptés au polymère de support. On peut citer par exemple les produits commerciaux de la marque commerciale ADMER^{®} de la société Mitsui Chemical qui correspondent à des polypropylène ou polyéthylène modifiés avec un anhydride maléique. Un polyéthylène modifié avec un anhydride maléique est par exemple le produit ADMER^{™} NF408E.

Particulièrement, la couche adhésive intercalaire a une épaisseur comprise entre 5 et 50 µm. Plus particulièrement, la couche adhésive intercalaire adhésive intercalaire a une épaisseur comprise entre 5 et 40 µm, entre 5 et 30 µm, entre 5 et 20 µm, entre 5 et 15 µm, et de préférence d'environ 10 µm.

Selon un mode préféré de l'invention, le conditionnement comprend deux couches adhésives intercalaires ayant chacune une épaisseur comprise entre 5 et 50 µm, de préférence d'environ 10 µm, lesdites deux couches adhésives intercalaires étant respectivement situées entre la couche interne de polypropylène et la couche centrale d'agent barrière aux gaz, et entre la couche externe de polyéthylène et la couche centrale d'agent barrière aux gaz.

Dans le cadre de la présente demande, une couche adhésive est aussi représentée par le sigle « / ». Un conditionnement selon l'invention comprenant au moins une couche adhésive peut ainsi être représenté selon les manières suivantes en fonction de la présence ou non de la couche centrale d'agent barrière aux gaz comprenant de l'éthylène-alcool vinylique : PP / PE, et PP / EVOH / PE.

Un autre conditionnement préféré de l'invention pour la conservation d'une composition pharmaceutique stérile comprend au moins les couches suivantes :
- une couche interne de polypropylène ayant une épaisseur d'environ 500 µm,
- une couche adhésive intercalaire ayant une épaisseur d'environ 10 µm, et
- une couche externe de polyéthylène ayant une épaisseur d'environ 600 µm.

Un autre conditionnement préféré de l'invention pour la conservation d'une composition pharmaceutique stérile comprend au moins les couches suivantes :
- une couche interne de polypropylène ayant une épaisseur d'environ 500 µm,
- une couche adhésive intercalaire ayant une épaisseur d'environ 10 µm,
- une couche centrale comprenant de l'éthylène vinyle alcool ayant une épaisseur d'environ 30 µm,
- une couche adhésive intercalaire ayant une épaisseur d'environ 10 µm, et
- une couche externe de polyéthylène basse densité ayant une épaisseur d'environ 600 µm.
Ce conditionnement préféré est représenté de la manière suivante : « PP (500 µm) / EVOH (30 µm) / PEBD (600 µm) » dans les exemples ci-après.

Un autre conditionnement préféré de l'invention pour la conservation d'une composition pharmaceutique stérile comprend au moins les couches suivantes :
- une couche interne de polypropylène ayant une épaisseur d'environ 500 µm,
- une couche adhésive intercalaire ayant une épaisseur d'environ 10 µm,
- une couche centrale comprenant de l'éthylène vinyle alcool ayant une épaisseur d'environ 50 µm,
- une couche adhésive intercalaire ayant une épaisseur d'environ 10 µm, et
- une couche externe de polyéthylène basse densité ayant une épaisseur d'environ 600 µm.
Ce conditionnement préféré est représenté de la manière suivante : « PP (500 µm) / EVOH (50 µm) / PEBD (600 µm) ».

### Composition

Un autre aspect de l'invention concerne un conditionnement, tel que décrit dans la présente demande, contenant en outre une composition pharmaceutique stérile.

Par « composition pharmaceutique » on entend toute composition pharmaceutique comprenant un principe actif. Une composition pharmaceutique inclut également les compositions vétérinaires. Selon un mode préféré de l'invention, la composition pharmaceutique est une composition vétérinaire.

Un principe actif comprend de manière générale les agents thérapeutiques et pharmaceutiques, les agents prophylactiques, les agents de diagnostic, et tout autre agent susceptible de prévenir et/ou traiter ou diagnostiquer une pathologie, une infection, une maladie chez des humains ou des animaux. Plus particulièrement, un principe actif inclut les antibiotiques, en particulier, les β-lactamines telles que l'amoxicilline, les céphalosporines telles que la céfalexine, et les tétracyclines telles que les oxytétracyclines, les agents anti-infectieux, les agents anti-coccidiens tels que le diclazuril et le toltrazuril, les vaccins tels que des vaccins comprenant par exemple des anticorps, des fragments d'anticorps, des peptides, des protéines, un interféron, une interleukine, une cytokine, et les vitamines, les agents anti-inflammatoires non stéroïdiens tels que le meloxicam et l'indomethacine, les agents anthelmintiques et antiparasitaires tels que le praziquantel, le pyrantel, l'ivermectine, la perméthrine, le fipronil, et le dinotefuran, les régulateurs de la croissance des insectes, les agents antiviraux, les agents cardiotoniques tels que la digoxine, les agents hypertenseurs, les agents diurétiques, les agents thérapeutiques pour le traitement de l'insuffisance cardiaque tels que le spironolactone et les inhibiteurs de l'enzyme de conversion de l'angiotensine tels que le benazépril, les agents d'induction et de synchronisation de l'oestrus et de l'ovulation tels que la buséréline. Un principe actif inclut également les agents de diagnostic qui peuvent être des polypeptides, des acides nucléiques, des polysaccharides, des lipides, des glycoprotéines, des glycolipides, des carbohydrates, des agents permettant de marquer certains tissus, tels que des agents contenant des radioisotopes, des marqueurs magnétiques, des marqueurs fluorescents, des marqueurs chimioluminescents, et des marqueurs enzymatiques.

De manière préférentielle, la composition pharmaceutique stérile comprend un principe actif choisi parmi l'amoxicilline, la buséréline, le toltrazuril, le diclazuril, ou la céfalexine.

Par « composition pharmaceutique stérile » on entend toute composition pharmaceutique décrite ci-dessus qui a été mise en oeuvre dans un procédé de stérilisation visant à détruire tout germe microbien. Ces procédés de stérilisation incluent notamment les traitements thermiques à la chaleur sèche ou à la chaleur humide (autoclavage), les traitements chimiques tels que les traitements utilisant l'oxyde d'éthylène, et les traitements par rayonnements UVs ou par rayonnements ionisants tels que ceux par exposition à un rayonnement gamma ou béta ou à un faisceau d'électrons accélérés.

La composition pharmaceutique stérile peut se présenter sous tout type de forme. Par exemple, la composition pharmaceutique stérile peut être une composition liquide, en particulier une composition aqueuse ou non-aqueuse. Un exemple de composition non-aqueuse peut être une composition huileuse. La composition pharmaceutique stérile peut être aussi une composition solide telle qu'une poudre, un comprimé, une capsule, un granule, une dragée, une gélule, un cachet, une pilule, une tablette, ou une pâte. De préférence, la composition pharmaceutique stérile est une composition aqueuse ou non-aqueuse. D'une manière encore plus préférée, la composition pharmaceutique non-aqueuse stérile est une composition huileuse.

Selon l'invention, le conditionnement convient ou est adapté à la conservation d'une composition pharmaceutique stérile. Selon un mode particulier, le conditionnement a une structure en trois dimensions (structure 3D). Ce mode particulier en structure 3D exclut donc les conditionnements sous forme de films, en particulier les films polymériques mono et multi-couches. Selon un mode préféré de l'invention, le conditionnement est une bouteille ou un flacon. Selon un mode encore plus préféré de l'invention, le conditionnement est un flacon pouvant contenir un volume de composition pharmaceutique stérile de 10 mL, 25 mL, 50 mL, 100 mL, 250 mL, 500 mL, 1 L, 2 L, de préférence 50 mL, 100 mL, 250 mL ou 500 mL.

Un autre objet de l'invention porte également sur une utilisation d'un conditionnement tel que décrit dans la présente demande pour conserver une composition pharmaceutique stérile.

Par conservation d'une composition pharmaceutique stérile dans le conditionnement selon l'invention, on peut entendre le maintien au cours du temps des propriétés physiques et chimiques de la composition pharmaceutique stérile, mais aussi celles du conditionnement. En effet, une dégradation du conditionnement au cours du temps altérerait par la même occasion la composition pharmaceutique, stérile à l'origine, contenue dans ce conditionnement. Ainsi, le conditionnement selon l'invention permet de conserver durablement ou sur le long-terme une composition pharmaceutique stérile. Plus spécifiquement, le conditionnement selon l'invention permet de conserver une composition pharmaceutique stérile pendant au moins 2, 3, 4, 5, 6, 7, 8, 9, 10, 11, 12, 15, 18, 24 mois, 3 ans, 4 ans, 5 ans, et plus. De préférence, le conditionnement selon l'invention permet de conserver une composition pharmaceutique stérile pendant au moins 6 mois, de préférence pendant au moins 12 mois, 24 mois, ou 36 mois.

Concernant les propriétés physiques et chimiques du conditionnement, celles-ci sont étudiées au cours du temps en les comparant généralement à un conditionnement en verre, qui est le conditionnement de référence couramment utilisé par l'homme du métier. Les paramètres physiques et chimiques du conditionnement étudiés sont, par exemple, les paramètres issus de l'analyse enthalpique différentielle qui permet d'avoir accès aux caractéristiques thermiques du conditionnement liées au changement d'état physique (fusion, cristallisation, transition vitreuse) ou chimique (réticulation, oxydation). Il peut être également étudié les propriétés mécaniques des flacons par un simple test de chute tel que détaillé dans les exemples ci-après.

Concernant les propriétés physiques et chimiques de la composition pharmaceutique stérile, celles-ci peuvent s'apprécier de manière absolue et sont régies par des règlementations ou pharmacopées qui définissent les paramètres physiques et chimiques devant être pris en compte et indiquent dans quelle mesure ces paramètres peuvent varier. L'évolution dans le temps de ces paramètres permet d'apprécier la stabilité physique et chimique des compositions pharmaceutiques stériles, et plus généralement, leur conservation dans le temps. Les paramètres peuvent être soient qualitatifs ou quantitatifs. Les paramètres qualitatifs pouvant être pris en compte sont notamment la couleur, la transparence, et l'odeur de la composition. Les paramètres quantitatifs sont, par exemple, le dosage ou le titrage d'un principe actif dans la composition, le pourcentage relatif de produit de dégradation par rapport au principe actif, le pH, et la viscosité. Généralement, dans le domaine pharmaceutique, les mesures du titrage du principe actif et du pourcentage relatif de produit de dégradation par rapport au principe actif sont suffisantes à la détermination de la stabilité de la composition pharmaceutique dans le temps. Ainsi, on considère une composition comme étant stable lorsque la teneur en principe actif dans la composition et le pourcentage relatif de produits de dégradation varient dans des proportions telles que l'efficacité et l'innocuité de la composition ne sont pas significativement modifiées. Les mesures peuvent être réalisées à différents temps, par exemple 3, 6, 12, 18, et 24 mois, à différentes températures, par exemple 4°C, 25°C, et 40°C, et à différentes conditions d'humidité. Plus particulièrement, il peut être considéré qu'une composition est stable lorsque la variation de la teneur en principe actif n'excède pas les 10 %, et préférentiellement les 5 %. Il peut être également considéré qu'une composition est stable lorsque la variation du pourcentage relatif de produits de dégradation est au plus de 10 %, de préférence au plus de 5 %. Ces intervalles sont notamment recommandés par la Pharmacopée Européenne ou décrits dans la Directive VICH GL3. Plus généralement, il est considéré que la durée de conservation et la stabilité d'une composition pharmaceutique sera d'autant plus longue que la variation du titre du principe actif au cours du temps sera faible et que l'apparition de produits de dégradation sera lente.

### Procédé

Un autre aspect de l'invention concerne un procédé pour la conservation stérile d'une composition pharmaceutique. La conservation stérile de la composition peut être obtenue selon deux modes particuliers.

Dans un premier mode particulier, une composition pharmaceutique est d'abord ajoutée dans un conditionnement tel que décrit ci-dessus. L'ensemble conditionnement/composition pharmaceutique est ensuite stérilisé par tout type de procédé connu de l'homme du métier.

Un objet de l'invention concerne donc un procédé pour la conservation stérile d'une composition pharmaceutique comprenant les étapes suivantes :
a) ajouter une composition pharmaceutique dans un conditionnement tel que décrit ci-dessus ; et
b) stériliser le conditionnement contenant la composition pharmaceutique.

Le procédé selon l'invention permet ainsi la stérilisation et la conservation stérile de la composition pharmaceutique.

Dans un second mode particulier, une composition pharmaceutique et un conditionnement tel que décrit ci-dessus sont stérilisés séparément. La composition pharmaceutique stérile est ensuite introduite dans le conditionnement stérile dans des conditions stériles.

Un objet de l'invention concerne donc un procédé pour la conservation stérile d'une composition pharmaceutique comprenant les étapes suivantes :
a) stériliser séparément un conditionnement tel que décrit ci-dessus et une composition pharmaceutique de manière à obtenir séparément un conditionnement stérile et une composition pharmaceutique stérile ; et
b) ajouter ladite composition pharmaceutique stérile dans ledit conditionnement stérile.

Les procédés décrits ci-dessus pour la conservation stérile d'une composition pharmaceutique peuvent comprendre en outre une étape optionnelle c) de conservation du conditionnement contenant la composition pharmaceutique stérile, de préférence dans des conditions adaptées à une bonne conservation d'une composition pharmaceutique. Par exemple, cette étape c) de conservation peut être réalisée à l'abri de la lumière, dans un environnement sec, à des températures n'excédant pas 20°C, sous vide, ces différentes conditions pouvant bien sûr être combinées les unes avec les autres.

L'étape b) de stérilisation mise en oeuvre dans les procédés décrits ci-dessus peut être réalisée par tout type de méthode de stérilisation connue de l'homme du métier. En particulier, l'étape b) de stérilisation peut être réalisée par chaleur humide, par chaleur sèche, par stérilisation gazeuse, et par irradiation. Selon un mode préféré, l'étape b) est réalisée par irradiation, telle que par irradiation gamma ou beta, par faisceau d'électron, et de préférence par irradiation gamma. Selon un mode encore plus préféré, l'étape b) est réalisée par irradiation gamma à une dose comprise entre 15 et 40 kGy, ou de préférence à 15, 25, 40 kGy, et d'une manière encore plus préférée à 25 ou 40 kGy.

Un procédé préféré de l'invention pour la conservation stérile d'une composition pharmaceutique comprend donc les étapes suivantes :
a) ajouter une composition pharmaceutique dans un conditionnement tel que décrit ci-dessus ; et
b) stériliser le conditionnement contenant la composition pharmaceutique par irradiation gamma, de préférence à une dose comprise entre 15 et 40 kGy.

D'autres aspects et avantages de l'invention apparaîtront à la lecture des exemples qui suivent, qui doivent être considérés comme illustratifs et non limitatifs.

### EXEMPLES

### Exemple 1 : test de chute

### Protocole :

Des échantillons de 20 flacons de 100 mL ont été traités ou non par irradiation gamma. Les échantillons ont ensuite été remplis d'eau et ont subi un test de chute en position verticale d'une hauteur de 1 mètre et 40 centimètres sans ou après irradiation. Pour les flacons irradiés, le test de chute a été réalisé juste après traitement d'irradiation ou après plusieurs mois de stockage à vide et à température ambiante.

### Résultats :

Les résultats sur les différents échantillons de flacons sont représentés dans le tableau 1 ci-après.

**Tableau 1 :**

| Composition des couches du flacon | Conditions d'irradiations | Nombre de flacons cassés sur 20 (Donnée moyenne) |
|---|---|---|
| **Flacon selon l'invention :** | sans irradiation | 0 |
| | 15-25 kGy | 0 |
| | 15-25 kGy et 12 mois de stockage | 0 |
| **PP (500 µm) / EVOH (30 µm) / PEBD (600 µm)** | 25 kGy | 0 |
| | 25 kGy et 12 mois de stockage | 0 |
| | 40 kGy | 0 |
| | 40 kGy et 12 mois de stockage | 1 |
| **Flacon selon l'invention :** | sans irradiation | 0 |
| **PP (480 µm) / EVOH (40 µm) / PEBD (560 µm)** | 40 kGy | 0 |
| | 40 kGy et 3 mois de stockage | 3 |
| **Flacon selon l'invention :** | sans irradiation | 0 |
| **PP (470 µm) / EVOH (40 µm) / PEBD + DYE (530 µm)** | 40 kGy | 0 |
| | 40 kGy et 3 mois de stockage | 0 |
| **Flacon selon l'invention :** | sans irradiation | 0 |
| **PP (490 µm) / EVOH (40 µm) / PEBD + DYE (520 µm)** | 40 kGy | 0 |
| | 40 kGy et 3 mois de stockage | 1 |
| Flacon comparatif : | sans irradiation | 0 |
| PP (500 µm) / EVOH (30 µm) / PEBD (200 µm) | 15-25 kGy | 3 |
| | 25 kGy | 15 |
| | 40 kGy | 17 |
| Flacon comparatif : | sans irradiation | 0 |
| PP (600 µm) / EVOH (100 µm) / PEBD (250 µm) | 40 kGy | 1 |
| | 40 kGy et 12 mois de stockage | 13 |

| | | |
|---|---|---|
| PP : Polypropylène EVOH : Ethylène vinyl alcool PEBD : Polyéthylène basse densité | | |

Les résultats du tableau 1 montrent que les flacons de l'invention comprenant une couche externe polymérique en PEBD d'une épaisseur moyenne de 600 µm sont résistants aux différentes doses d'irradiation, et ce jusqu'à 40 kGy et après 12 mois de stockage (un seul flacon sur vingt flacons se casse dans les conditions les plus extrêmes).

Au contraire, la majorité des flacons comprenant une couche fine de PEBD de 200 µm se cassent juste après avoir été irradiée à 25 kGy (15/20) et à 40 kGy (17/20). En outre, un nombre important de flacons (13/20) comprenant une couche fine de PEBD de 250 µm se casse dans les conditions les plus extrêmes (irradiation 40 kGy et 12 mois de stockage).

Ces résultats démontrent qu'une couche externe polymérique en PE d'une épaisseur supérieure à 250 µm, en particulier comprise entre 300 et 1000 µm, est nécessaire pour fournir un conditionnement résistant à de fortes doses d'irradiation, permettant ainsi de conserver une composition pharmaceutique stérile pendant une longue durée (au moins 12 mois).

### Exemple 2 : Etude de l'effet barrière aux gaz et à l'eau

### Protocole étude de perméabilité à l'eau :

Des flacons irradiés ou non irradiés selon l'invention ont été remplis de 10 g de tamis moléculaire, puis bouchés hermétiquement, pesés et placés en enceinte climatique à 40°C/75% HR pendant environ 3 mois.

Ils ont été régulièrement pesés tout au long de cette période pour mesurer leur prise de poids qui permet d'évaluer la capacité du conditionnement à laisser pénétrer de la vapeur d'eau.

### Protocole étude de perméabilité à l'oxygène :

Les mesures de perméabilité à l'oxygène sur les flacons irradiés ou non irradiés selon l'invention ont été réalisées selon la norme ASTM F2622.

### Résultats :

Les résultats sur les différents échantillons de flacons sont représentés dans le tableau 2 ci-après.

**Tableau 2 :**

| **Composition des couches du flacon selon l'invention** | **Conditions d'irradiations** | **Coefficient moyen de transmission de la vapeur d'eau (g/24 h à 40 °C + 75% HR)** | **Oxygène transmission (cm3/24h)** |
|---|---|---|---|
| **PP (500 µm)** / **EVOH (30 µm)** / **PEBD (600 µm)** | Non irradié | 0,0026 | 0,0013 |
| | 15 kGY | 0,0035 | - |
| | 25 kGy | 0,0035 | 0,0015 |
| | 40kGy | 0,0032 | 0,0011 |
| **PP (500 µm)** / **EVOH (30 µm)** / **PEBD (600 µm)** | 25 kGy | ND | 0,0021 |
| **Stockés à TA 8 mois après traitement** | 40kGy | ND | 0,0025 |
| **PP (470 µm) / EVOH (30 µm) / PEBD + DYE (530 µm)** | 40kGy | 0,003 | ND |
| **PP (490 µm) / EVOH (40 µm)** / **PEBD Purell 3020D + DYE (520 µm)** | Non irradié | ND | 0,002 |
| | 40kGy | 0,0027 | 0,0020 |

| | | | |
|---|---|---|---|
| PP : Polypropylène EVOH : Ethylène vinyl alcool PEBD : Polyéthylène basse densité HR : Humidité relative TA : Température ambiante ND : Non Déterminé | | | |

Les résultats du tableau 2 montrent que l'irradiation gamma n'a aucun impact sur les propriétés barrières à l'oxygène ni à la vapeur d'eau des conditionnements de l'invention.

### Exemple 3 : Etude de la stabilité de la composition pharmaceutique

### Protocole :

Des flacons selon l'invention (PP (500 µm) / EVOH (30 µm) / PEBD (600 µm) ont été remplis avec une suspension d'amoxicilline puis irradiés par irradiation gamma à 4 doses différentes (7, 15, 25, et 40 kGy). Ces flacons ont ensuite été analysés avant et après stockage pendant 6 mois à 40°C/75% HR.

### Résultats :

Les résultats sur les différents échantillons de flacons sont représentés dans le tableau 3 ci-après.

**Tableau 3 :**

| Conditions d'irradiations | Aspect du produit | Dosage Amoxicilline (mg/mL) avant stockage | Dosage Amoxicilline (mg/mL) après T6 mois de stockage à 40°C/75%HR | Dosage des produits de dégradation Totaux (%) |
|---|---|---|---|---|
| Non irradié | suspension blanc crème | 200 | - | 1,2 |
| Non irradié | suspension blanc crème | 200 | 195 | 2,0 |
| 7 kGy | suspension beige crème | 200 | 193 | 2,1 |
| 15 kGy | suspension brun pâle crème | 200 | 193 | 2,2 |
| 25 kGy | suspension brun pâle crème | 200 | 193 | 2,3 |
| 40 kGy | suspension brun pâle crème | 200 | 191 | 2,2 |
| 7 kGy | suspension beige crème | 200 | 195 | 2,2 |
| 15 kGy | suspension brun pâle crème | 200 | 195 | 2,2 |
| 25 kGy | suspension brun pâle crème | 200 | 194 | 2,0 |
| 40 kGy | suspension brun pâle crème | 200 | 193 | 2,1 |

La première ligne du tableau 3 correspond au dosage de l'amoxicilline et des produits de dégradation à T0, c'est-à-dire sans stockage.

Les résultats du tableau 3 montrent que l'étape d'irradiation, quel que soit la dose, n'impacte pas la stabilité de la suspension d'amoxicilline.

En effet, la concentration en principe actif reste dans la fourchette acceptable pour un produit pharmaceutique de +/- 5 % et la quantité de produits de dégradation observée n'excède pas ce qui est acceptable.

Les inventeurs ont démontré que les conditionnements selon l'invention après irradiation permettaient de conserver la stabilité d'un produit pharmaceutique stocké dans des conditions accélérées (40°C/75% HR) pendant 6 mois. Ces résultats obtenus dans ces conditions accélérés garantissent une stabilité du produit pharmaceutique d'au moins 18 mois à température ambiante.

## Revendications

1. Conditionnement pour la conservation d'une composition pharmaceutique stérile comprenant au moins une couche interne de polypropylène ayant une épaisseur comprise entre 200 et 800 µm et une couche externe de polyéthylène, ladite couche externe étant en contact avec l'environnement et ayant une épaisseur comprise entre 300 et 1000 µm.

2. Conditionnement selon la revendication 1, dans lequel la couche interne de polypropylène a une épaisseur comprise entre 300 et 700 µm, entre 350 et 650 µm, entre 400 et 600 µm, de préférence entre 450 et 550 µm, et d'une manière encore plus préférée d'environ 500 µm.

3. Conditionnement selon la revendication 1 ou 2, dans lequel la couche externe de polyéthylène a une épaisseur comprise entre 400 et 800 µm, entre 450 et 750 µm, entre 500 et 700 µm, de préférence entre 550 et 650 µm, et d'une manière encore plus préférée d'environ 600 µm.

4. Conditionnement selon l'une quelconque des revendications 1 à 3, dans lequel la couche externe de polyéthylène comprend en outre au moins un colorant anti-UV.

5. Conditionnement selon l'une quelconque des revendications 1 à 4, dans lequel le conditionnement comprend en outre une couche centrale d'agent barrière aux gaz située entre la couche interne de polypropylène et la couche externe de polyéthylène, et ayant une épaisseur comprise entre 10 et 250 µm, entre 10 et 200 µm, entre 20 et 80 µm, de préférence entre 20 et 50 µm, et d'une manière encore plus préférée d'environ 30 ou 50 µm.

6. Conditionnement selon la revendication 5, dans lequel la couche centrale d'agent barrière aux gaz comprend de l'éthylène-alcool vinylique.

7. Conditionnement selon l'une des revendications 5 ou 6 dans lequel le conditionnement comprend au moins les couches suivantes :
- une couche interne de polypropylène ayant une épaisseur d'environ 500 µm,
- une couche centrale comprenant de l'éthylène vinyle alcool ayant une épaisseur d'environ 30 ou 50 µm, et
- une couche externe de polyéthylène ayant une épaisseur d'environ 600 µm.

8. Conditionnement selon l'une quelconque des revendications 5 à 7, dans lequel le conditionnement comprend deux couches adhésives intercalaires ayant chacune une épaisseur comprise entre 5 et 50 µm, de préférence d'environ 10 µm, lesdites deux couches adhésives intercalaires étant respectivement situées entre la couche interne de polypropylène et la couche centrale d'agent barrière aux gaz, et entre la couche externe de polyéthylène et la couche centrale d'agent barrière aux gaz.

9. Conditionnement selon l'une quelconque des revendications 1 à 8, dans lequel le polyéthylène est un polyéthylène basse densité.

10. Conditionnement selon l'une quelconque des revendications 1 à 9, contenant en outre une composition pharmaceutique stérile, de préférence aqueuse ou non-aqueuse, en particulier huileuse.

11. Conditionnement selon l'une quelconque des revendications 1 à 10, dans lequel la composition pharmaceutique stérile comprend un principe actif choisi parmi l'amoxicilline, la buséréline, le toltrazuril, le diclazuril, ou la céfalexine.

12. Conditionnement selon l'une quelconque des revendications 1 à 11, dans lequel le conditionnement est une bouteille ou un flacon.

13. Utilisation d'un conditionnement tel que défini selon l'une quelconque des revendications 1 à 12 pour conserver une composition pharmaceutique stérile.

14. Procédé pour la conservation stérile d'une composition pharmaceutique comprenant les étapes suivantes :
a) ajouter une composition pharmaceutique dans un conditionnement tel que défini selon l'une quelconque des revendications 1 à 12 ; et
b) stériliser le conditionnement contenant la composition pharmaceutique.

15. Procédé selon la revendication 14, dans lequel le conditionnement contenant la composition pharmaceutique à l'étape b) est stérilisé par irradiation gamma, de préférence à une dose comprise entre 15 et 40 kGy.

## Patentansprüche

1. Verpackung zum Konservieren einer sterilen pharmazeutischen Zusammensetzung, umfassend mindestens eine innere Schicht aus Polypropylen mit einer Dicke zwischen 200 und 800 µm und eine äußere Schicht aus Polyethylen, wobei die äußere Schicht in Kontakt mit der Umgebung steht und eine Dicke zwischen 300 und 1.000 µm aufweist.

2. Verpackung nach Anspruch 1, wobei die innere Polypropylenschicht eine Dicke zwischen 300 und 700 µm, zwischen 350 und 650 µm, zwischen 400 und 600 µm, vorzugsweise zwischen 450 und 550 µm und noch mehr bevorzugt von etwa 500 µm, aufweist.

3. Verpackung nach Anspruch 1 oder 2, wobei die äußere Polyethylenschicht eine Dicke zwischen 400 und 800 µm, zwischen 450 und 750 µm, zwischen 500 und 700 µm, vorzugsweise zwischen 550 und 650 µm und noch mehr bevorzugt von etwa 600 µm, aufweist.

4. Verpackung nach einem der Ansprüche 1 bis 3, wobei die äußere Polyethylenschicht ferner mindestens einen Anti-UV-Farbstoff umfasst.

5. Verpackung nach einem der Ansprüche 1 bis 4, wobei die Verpackung ferner eine mittlere Schicht aus einem Gasbarrieremittel umfasst, die sich zwischen der inneren Polypropylenschicht und der äußeren Polyethylenschicht befindet und eine Dicke zwischen 10 und 250 µm, zwischen 10 und 200 µm, zwischen 20 und 80 µm, vorzugsweise zwischen 20 und 50 µm und noch mehr bevorzugt von etwa 30 oder 50 µm, aufweist.

6. Verpackung nach Anspruch 5, wobei die mittlere Schicht des Gasbarrieremittels Ethylenvinylalkohol umfasst.

7. Verpackung nach einem der Ansprüche 5 oder 6, wobei die Verpackung mindestens die folgenden Schichten umfasst:
- eine innere Polypropylenschicht mit einer Dicke von etwa 500 µm,
- eine mittlere Schicht, die Ethylenvinylalkohol umfasst, mit einer Dicke von etwa 30 oder 50 µm und
- eine äußere Polyethylenschicht mit einer Dicke von etwa 600 µm.

8. Verpackung nach einem der Ansprüche 5 bis 7, wobei die Verpackung zwei Zwischenklebstoffschichten umfasst, die jeweils eine Dicke zwischen 5 und 50 µm, vorzugsweise von etwa 10 µm, aufweisen, wobei die zwei Zwischenklebstoffschichten sich jeweils zwischen der inneren Polypropylenschicht und der mittleren Schicht aus Gasbarrieremittel und zwischen der äußeren Polyethylenschicht und der mittleren Schicht aus Gasbarrieremittel befinden.

9. Verpackung nach einem der Ansprüche 1 bis 8, wobei das Polyethylen ein Polyethylen mit geringer Dichte ist.

10. Verpackung nach einem der Ansprüche 1 bis 9, die ferner eine sterile pharmazeutische Zusammensetzung enthält, die vorzugsweise wässrig oder nichtwässrig, insbesondere ölig ist.

11. Verpackung nach einem der Ansprüche 1 bis 10, wobei die sterile pharmazeutische Zusammensetzung einen Wirkstoff enthält, der aus Amoxicillin, Buserelin, Toltrazuril, Diclazuril oder Cefalexin ausgewählt ist.

12. Verpackung nach einem der Ansprüche 1 bis 11, wobei die Verpackung eine Flasche oder ein Fläschchen ist.

13. Verwendung einer Verpackung wie nach einem der Ansprüche 1 bis 12 zum Konservieren einer sterilen pharmazeutischen Zusammensetzung.

14. Verfahren zum sterilen Konservieren einer pharmazeutischen Zusammensetzung, umfassend die folgenden Schritte:
a) Hinzufügen einer pharmazeutischen Zusammensetzung zu einer Verpackung nach einem der Ansprüche 1 bis 12; und
b) Sterilisieren der Verpackung, die die pharmazeutische Zusammensetzung enthält.

15. Verfahren nach Anspruch 14, wobei die Verpackung, die die pharmazeutische Zusammensetzung enthält, in Schritt b) durch Gammabestrahlung, vorzugsweise mit einer Dosis zwischen 15 und 40 kGy, sterilisiert wird.

## Claims

1. Packaging for preserving a sterile pharmaceutical composition, comprising at least one inner layer of polypropylene having a thickness of between 200 and 800 µm and one outer layer of polyethylene, said outer layer being in contact with the environment and having a thickness of between 300 and 1000 µm.

2. Packaging according to claim 1, wherein the inner layer of polypropylene has a thickness of between 300 and 700 µm, between 350 and 650 µm, between 400 and 600 µm, preferably between 450 and 550 µm, and even more preferably of approximately 500 µm.

3. Packaging according to either claim 1 or claim 2, wherein the outer layer of polyethylene has a thickness of between 400 and 800 µm, between 450 and 750 µm, between 500 and 700 µm, preferably between 550 and 650 µm, and even more preferably of approximately 600 µm.

4. Packaging according to any of claims 1 to 3, wherein the outer layer of polyethylene further comprises at least one anti-UV colorant.

5. Packaging according to any of claims 1 to 4, wherein the packaging further comprises a central layer of gas barrier agent that is located between the inner layer of polypropylene and the outer layer of polyethylene, and having a thickness of between 10 and 250 µm, between 10 and 200 µm, between 20 and 80 µm, preferably between 20 and 50 µm, and even more preferably of approximately 30 or 50 µm.

6. Packaging according to claim 5, wherein the central layer of gas barrier agent comprises ethylene vinyl alcohol.

7. Packaging according to either claim 5 or claim 6, wherein the packaging comprises at least the following layers:
- an inner layer of polypropylene having a thickness of approximately 500 µm,
- a central layer comprising ethylene vinyl alcohol having a thickness of approximately 30 or 50 µm, and
- an outer layer of polyethylene having a thickness of approximately 600 µm.

8. Packaging according to any of claims 5 to 7, wherein the packaging comprises two adhesive interlayers each having a thickness of between 5 and 50 µm, preferably of approximately 10 µm, said two adhesive interlayers being located between the inner layer of polypropylene and the central layer of gas barrier agent, and between the outer layer of polyethylene and the central layer of gas barrier agent, respectively.

9. Packaging according to any of claims 1 to 8, wherein the polyethylene is a low density polyethylene.

10. Packaging according to any of claims 1 to 9, further containing a sterile pharmaceutical composition which is preferably aqueous or non-aqueous, in particular oily.

11. Packaging according to any of claims 1 to 10, wherein the sterile pharmaceutical composition comprises an active ingredient selected from amoxicillin, buserelin, toltrazuril, diclazuril, or cefalexin.

12. Packaging according to any of claims 1 to 11, wherein the packaging is a bottle or a vial.

13. Use of a packaging as defined according to any of claims 1 to 12 for preserving a sterile pharmaceutical composition.

14. Method for the sterile preservation of a pharmaceutical composition, comprising the following steps:
a) adding a pharmaceutical composition into a packaging as defined according to any of claims 1 to 12; and
b) sterilizing the packaging containing the pharmaceutical composition.

15. Method according to claim 14, wherein the packaging containing the pharmaceutical composition in step b) is sterilized by gamma irradiation, preferably at a dose of between 15 and 40 kGy.
